Europäisches Patentamt

European Patent Office

Office européen des brevets

(11) Publication number: **0 180 820**
**B1**

(12) **EUROPEAN PATENT SPECIFICATION**

(45) Date of publication of patent specification: **23.01.91**　　(51) Int. Cl.⁵: **A 61 B 17/064,** A 61 B 17/068

(21) Application number: **85113072.4**

(22) Date of filing: **15.10.85**

(54) Surgical closure element.

(30) Priority: **08.11.84 US 669497**

(43) Date of publication of application:
**14.05.86 Bulletin 86/20**

(45) Publication of the grant of the patent:
**23.01.91 Bulletin 91/04**

(84) Designated Contracting States:
**BE CH DE FR GB IT LI NL SE**

(56) References cited:
**EP-A-0 050 554**
**EP-A-0 154 815**
**US-A-2 910 067**
**US-A-3 209 754**
**US-A-3 753 438**
**US-A-4 014 492**
**US-A-4 043 504**
**US-A-4 399 810**

(73) Proprietor: **AMERICAN CYANAMID COMPANY**
**1937 West Main Street P.O. Box 60**
**Stamford Connecticut 06904-0060 (US)**

(72) Inventor: **Burbank, John Emerson, III**
**106 Haviland Road**
**Ridgefield Connecticut (US)**
Inventor: **Montgomery, John R.**
**715 Sasco Hill Road**
**Fairfield Connecticut (US)**

(74) Representative: **Wächtershäuser, Günter, Dr.**
**Tal 29**
**D-8000 München 2 (DE)**

Courier Press, Leamington Spa, England.

## Description

The present invention relates to a surgical stapler cartridge of a wound closure element of the type disclosed in non-prepublished EP—A—0 154 815 by the present applicant, which document falls under Art. 54(3) of the EPC. From US—A—4 043 504 the most relevant prepublished prior art is known.

The improvement allows for the sequential motion without jamming, of a plurality of closure elements in a feed track. In the feed track, the legs of an element are adjacent the crown of a preceding element. This invention specifically relates to a feed track containing two or more surgical wound closure elements. The invention can be useful in a ligating instrument containing a plurality of ligating clips and is useful in a surgical stapler containing a plurality of staples.

This invention decreases the frictional resistance between the plurality of closure elements and the feed track. An advantage of this is that the force required to linearly move a plurality of closure elements in the feed track is reduced. Another advantage is that a greater number of closure elements can be loaded into a surgical cartridge or magazine.

The improvement to the closure element cartridge enables the use of a very thin feed delivery system. This advantage cannot be overemphasized. Specifically, this advantage provides greater visibility of the crimping mechanism, e.g. an anvil for a surgical staple or a pair of jaws for a ligating clip, and therfore vastly improves, if not insures, proper placement of a closure element at the wound site.

This invention makes possible the feeding of a plurality of closure elements in a surgical instrument cartridge, e.g. a plurality of more than about twenty, which are sequentially in direct contact with one another in a leg to crown configuration.

It is an object of this invention to minimize friction in a feed track. The friction magnifies exponentially with the number of closure elements in the feed track.

A surgical instrument cartridge is described. The cartridge comprises a plurality of wound closure elements. Each element has a crown and two opposite side legs. The distal end of each leg of a wound closure element contacts the crown of the adjacent preceding element.

The instrument cartridge also comprises means for containing at least each end of each crown and the proximal end of each leg in the cartridge. Further, the cartridge comprises means for attaching the cartridge to a surgical instrument.

The improvement comprises the legs of each element being self-aligning in the containing means, relative to the adjacent preceding element.

One embodiment of the improvement comprises a deformation adjacent each end of each crown. The distal end of each leg of an element contacts the respective deformation of the adjacent preceding element. Alternatively or in combination with this embodiment, an embodiment of the improvement comprises the distal ends of the legs of each element being in an essentially converging relationship.

In a preferred embodiment, each element is a staple. In this preferred embodiment, the improvement comprises at least one bend in each crown of each staple. The distal ends of the legs of each staple are then in an essentially symmetrical converging relationship.

Description of the Drawings

Figures. 1 to 4 and 11 show prior art embodiments.

Figure 1 is a front view showing the legs of a wound closure element in a converging relationship;

Figure 2 is a cutaway side view taken on the plane 2—2 of Figure 1;

Figures 3, 5 and 7 are front views showing at least one bend in each crown of a wound closure element and the legs of each element in a converging relationship;

Figures 4, 6 and 8 are cutaway side views taken on the plane 4—4 of Figure 3, the plane 6—6 of Figure 5, and the plane 8—8 of Figure 7, respectively;

Figure 9 is a front view showing a deformation adjacent each end of the crown of a wound closure element;

Figure 10 is a cutaway side view taken on the plane 10—10 of Figure 9;

Figure 11 is a cutaway front view of a surgical instrument cartridge showing a plurality of wound closure elements, the distal end of each leg of a wound closure element contacting the crown of the adjacent preceding element; and

Figures 12 to 14 are alternative embodiments to the plurality of wound closure elements shown in Figure 11.

Description

A column of two or more wound closure elements being sequentially moved in a leg to crown fashion can employ this invention. The amount of convergence in the legs either independent of or combined with the amount of bend in the crown, can vary.

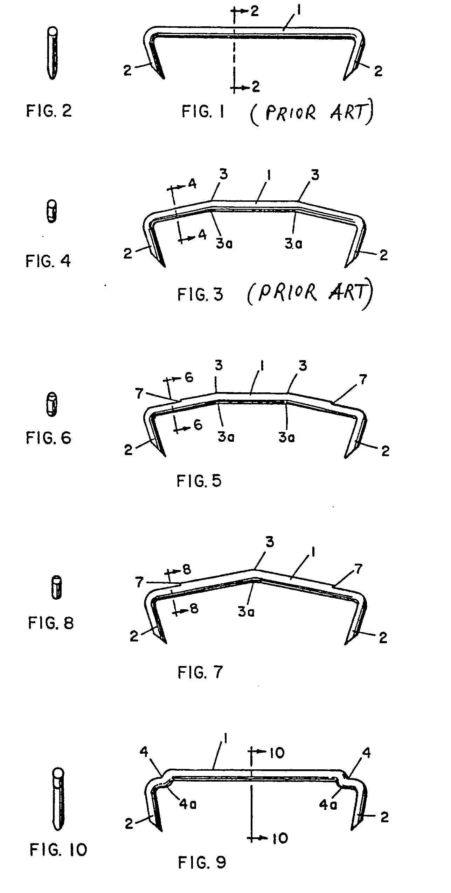

A bend in the closure element crown is not necessary, see for example Figures 9, 10 and 14, if the closure element legs can be otherwise deflected inward so they do not ride onto the arc between each end of the crown and each leg.

Side thrust is a component of forward thrust. It is created when one of the distal ends of a leg moves laterally. For a particular closure element, the side thrust is in direct proportion to the forward thrust. Side thrust can be calculated as a function of the imbalance between the two instantaneous slopes experienced by the distal ends of each leg (the slopes are relative to a plane which is perpendicular to the direction of the desired forward motion).

Side thrust by a closure element causes resistance to the forward motion of the column of closure elements. This is due to the friction between the closure element and the walls of the cartridge (or magazine) tracks. The amount of friction is the product of the side thrust multiplied by the coefficient of friction between the closure element and the tracks.

The force required to move the column of closure elements, as seen by any particular closure element, is increasing by the side thrust multiplied by the coefficient of friction. Since the side thrust is in direct proportion to the forward thrust, each closure element closer to the force necessary to move the column of closure elements, is resisting forward motion more than the preceding closure element. This is due to the increased forward thrust received. The total frictional loss of the column of closure elements in the cartridge tracks is the sum of the frictional losses of each closure element. When the total frictional loss is greater than or equal to the force necessary to move the column of closure elements, the most distal closure element cannot move.

Due to the above described exponential reaction possible with a plurality of closure elements, it is desirable to relieve the source of side thrust. One way to do this is to use closure elements whose ends are converging toward each other sufficiently so that they contact only a flat area or balanced slopes on the preceding closure element crown. Providing a bend to the preceding crown is one way of doing this. Providing a deformation adjacent each end of each closure element crown, such as notches, dents, steps or similar means for guiding is also a way. Relieving the source of side thrust allows more closure elements to be contained in cartridge.

The term wound closure element is intended to be generic and includes, but is not limited to, a staple, clip, clamp, fastener, pin or a similar closure element. A surgical staple is preferred. The wound closure element exemplified in Figures 1 to 14 is a surgical staple.

Referring to Figures 1 to 10, a surgical wound closure element has a crown 1. Two opposite side legs 2 are contained at each opposite end of the crown 1. The improvement of this invention comprises the distal ends of the opposite side legs 2 of each element being in an essentially converging relationship (Figures 1 and 2); at least one bend 3 in the crown (Figures 3 to 8 and 11 to 13); and a deformation 4 adjacent each end of each crown 1, the distal end of each leg 2 of an element contacting the respective deformation of the preceding element (Figures 9, 10 and 14).

The direction of the bend 3 can be in the direction of each side leg 2. The bend enables at least the distal ends of the legs 2 of each element to be in an essentially converging relationship.

Referring to Figures 3 to 6, 11 and 12, two bends 3 are essentially equidistant from the center of the crown 1.

Referring to Figures 7, 8 and 13, the bend 3 is essentially at about the center of the crown 1. Referring to Figures 5 to 8, 12 and 13, the notches 7 are about equidistance from the center of the crown 1. The notches act as a stop for the distal ends (which can be pointed) of an adjacent wound closure element.

Referring to Figures 3, 5, 7 and 11 to 13, the bend 3a on the underside of the crown 1 can be formed during the manufacture of the bend 3. It is to be understood that the bend 3a is not critical to the practice of this invention. That is, alternative wound closure elements may be manufactured having an essentially triangular shaped crown, wherein the lower portion of the crown is essentially planar.

Referring to Figures 9, 10 and 14, an alternative wound closure element is described. The wound closure element has a crown 1 and two opposite side legs 2. The improvement comprises a deformation 4 at about each end of the crown 1. At least the distal ends of each leg 2 can also be in an essentially converging relationship.

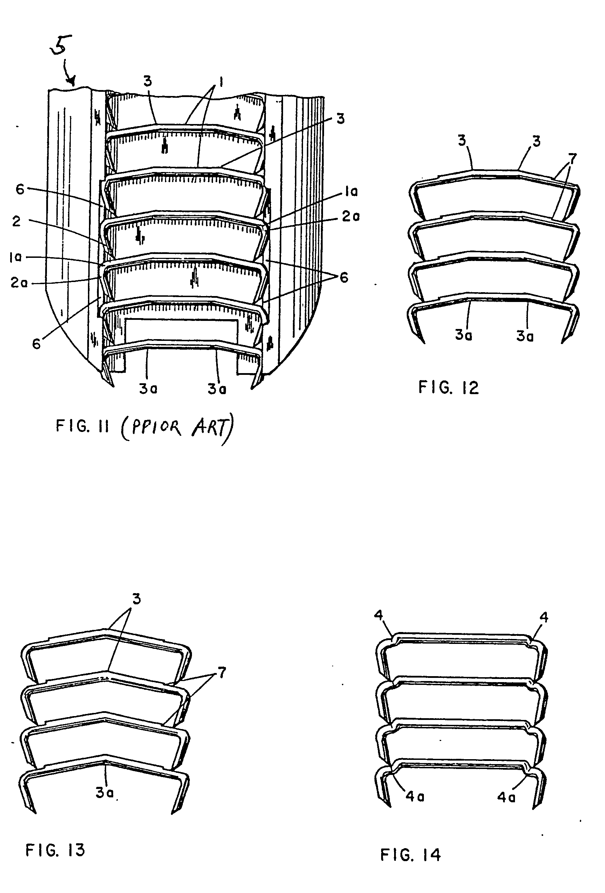

Referring to Figures 11 to 14, a surgical cartridge 5 is disclosed. The cartridge comprises a plurality of wound closure elements. The elements shown in Figures 11 to 14 are essentially a plurality of the elements shown in Figures 3, 5, 7 and 9, respectively.

Each wound closure element comprises a crown 1 and two opposite side legs 2. The relationship of each wound closure element to the adjacent element in the cartridge is a point to crown relationship. That is, at least the distal ends of the legs (which can be pointed) of a wound closure element contact the crown 1 of the preceding element. But for the point to crown relationship, each wound closure element is in an essentially noncontiguous relationship to the adjacent element.

Referring again to Figure 11, besides a plurality of wound closure elements, the surgical cartridge 5 comprises a means 6 for containing at least the ends 1a of each crown and the proximal ends 2a of each leg. Preferably, the contacting means are two opposite grooves. Although not shown, the cartridge 5 can also contain means for attaching the cartridge to a surgical instrument, and means for activating the plurality of closure elements by the surgical instrument. The attaching and the activating means are known in the prior art. Please see, for example, EP—A—0 154 815. The improvement to the cartridge of this invention allows the plurality of elements to be self-aligning in the containing means 6.

It is to be understood that the plurality of wound closure elements described in Figures 12 to 14 can be alternatively used in the cartridge 5 shown in Figure 11. That is, it is to be understood that the wound closure elements shown in Figures 12 to 14 are interchangeable with the wound closure elements shown in Figure 11.

In a final embodiment, the distal ends of the grooves 6 are adjacent to an anvil. A description of an anvil which can be used to practice this embodiment is disclosed in the prior art, for example the application described above.

## Claims

1. A surgical stapler cartridge (5) of the type having a staple feed track movably containing a plurality of surgical staples, means for attaching the cartridge to said surgical stapler, and means for activating said plurality of staples by the surgical stapler, each staple having a crown (1) and two opposed side legs (2), at least one bend (3) in the crown such that the distal ends of said legs (2) of each staple are in an essentially symmetrical converging relationship, and a deformation (4; 7) adjacent each end (1a) of said crown (1), the distal end of each leg (2) of each surgical staple contacting the respective deformation (4; 7) in the crown (1) of the adjacent preceding staple, whereby the legs of the plurality of staples are self-aligning in the staple feed track.

2. The cartridge of Claim 1 wherein said staple feed track comprises two opposed grooves.

3. The cartridge of Claim 1 wherein said crown (1) has two bends (3) equidistant from the center of said crown.

4. The cartridge of Claim 1 wherein said deformations (4; 7) provide an essentially flat surface for receiving the distal ends of each leg (2) of the adjacent following staple.

## Patentansprüche

1. Chirurgische Heftmaschinepatrone (5) der Art mit einem Klammernführungsweg, der mehrere von chirurgischen Klammern bewegbar enthält, Einrichtungen zum Befestigen der Patrone an der chirurgischen Heftmaschine, und Einrichtungen zum Aktivieren der mehreren von Klammern durch die chirurgische Heftmaschine, wobei jede Klammer ein Oberteil (1) und zwei gegenüberliegende Seitenschenkel (2) aufweist, sowie wenigstens einen Knick (3) in dem Oberteil, so daß die entfernten Enden der Schenkel (2) jede Klammer in einem im wesentlichen symmetrischen Verhältnis stehen, und eine Umformung (4; 7), die an jedem (1a) des Oberteils (1) angrenzt, wobei die entfernten Enden jedes Schenkels (2) jeder chirurgischen Klammer die einzelnen Umformungen (4; 7) in dem Oberteil (1) der angrenzenden, vorhergehenden Klammer kontaktieren, wodurch die Schenkel der mehreren von Klammern in dem Klammernführungsweg selbstausgerichtet werden.

2. Patrone nach Anspruch 1, worin der Klammernführungsweg zwei gegenüberliegende Nuten aufweist.

3. Patrone nach Anspruch 1, worin das Oberteil (2) zwei Knicke (3) aufweist, die abstandsgleich von der Mitte des Oberteils angeordnet sind.

4. Patrone nach Anspruch 1, worin die Umformungen (4; 7) eine im weentlichen flache Oberfläche bilden, um die entfernten Enden jedes Schenkels (2) der angrenzenden Klammer aufzunehmen.

## Revendications

1. Cartouche (5) pour agrafeuse chirurgicale du type comportant une glissière d'alimentation en agrafes contenant de façon mobile une multiplicité d'agrafes chirurgicales, des moyens de fixation de la cartouche à ladite agrafeuse chirurgicale et des moyens d'actionnement par l'agrafeuse chirurgicale de ladite multiplicité d'agrafes, chaque agrafe présentant une partie supérieure (1) et deux branches latérales opposées (2), au moins une courbure (3) prévue dans la partie supérieure (1) de telle sorte que les extrémités distales desdites branches (2) de chaque agrafe se trouvent dans une relation convergente sensiblement symétrique, et une déformation (4; 7) près de chaque extrémité (1a) de ladite partie supérieure (1), l'extrémité distale de chaque branche (2) de chaque agrafe chirurgicale étant en contact avec la déformation respective (4; 7) de la partie supérieure (1) de l'agrafe précédente adjacente, moyennant quoi les branches des agrafes s'alignent automatiquement dans la glissière d'alimentation en agrafes.

2. Cartouche selon la revendication 1, dans laquelle ladite glissière d'alimentation en agrafes comporte deux rainures opposées.

3. Cartouche selon la revendication 1, dans laquelle ladite partie supérieure (1) présente deux courbures (3) équidistantes du centre de ladite partie supérieure.

4. Cartouche selon la revendication 1, dans laquelle lesdites déformations (4; 7) définissent une surface sensiblement plane destinée à recevoir les extrémités distales de chaque branche (2) de l'agrafe suivante adjacent.

EP 0 180 820 B1

FIG. 2

FIG. 1 (PRIOR ART)

FIG. 4

FIG. 3 (PRIOR ART)

FIG. 6

FIG. 5

FIG. 8

FIG. 7

FIG. 10

FIG. 9

1

FIG. 11 (PPIOR ART)

FIG. 12

FIG. 13

FIG. 14